# EUROPÄISCHE PATENTSCHRIFT

(11) **EP 4 046 511 B1**
(45) Veröffentlichungstag und Bekanntmachung des Hinweises auf die Patenterteilung: **06.09.2023**
(21) Anmeldenummer: 22020053.9
(22) Anmeldetag: 17.02.2022
(51) Int. Cl.: A41C 3/00, A41C 5/00, A41D 27/24, A41H 43/04, B32B 7/14, A41D 13/015, A61F 13/08, B32B 3/08, B32B 5/26, B32B 7/05

(54) **VERFAHREN ZUR HERSTELLUNG VON BEKLEIDUNGSSTÜCKEN ODER BANDAGEN**
METHOD FOR FORMING GARMENTS OR BANDAGES
PROCÉDÉ DE FABRICATION DE VÊTEMENTS OU DE BANDAGES

(30) Priorität: 17.02.2021 DE 102021103737
(43) Veröffentlichungstag der Anmeldung: 24.08.2022
(73) Patentinhaber: NTT New Textile Technologies GmbH, 72336 Balingen (DE)
(72) Erfinder: BAUER, Hans, 72336 Balingen (DE)
(74) Vertreter: Müller, Gottfried

(56) Entgegenhaltungen:
- EP-B1- 2 822 520
- EP-B2- 1 211 956
- DE-A1-102017 119 328

## Beschreibung

Die Erfindung bezieht sich auf ein Verfahren zur Herstellung von Bekleidungsstücken oder Bandagen mit zwei übereinanderliegenden Stofflagen.

Aus der EP 2 621 296 B1 ist ein Bekleidungsstück mit zwei aufeinanderliegenden Gewebelagen bekannt, die über Klebstoff in Form eines Klebstoffmusters miteinander verbunden sind. Als Klebstoff wird ein Elastomer verwendet. Die innenliegende Seite einer der aufeinanderliegenden Gewebelagen ist mit Beflockungsmaterial versehen.

In der EP 1 211 956 B2 wird ein Verfahren zur Herstellung von Unterbekleidungsstücken beschrieben, bei dem eine obere und eine untere Stofflage durch elastomere Kunststoff-Klebeaufträge miteinander verbunden werden. Der Randbereich des Unterbekleidungsstücks wird durch die elastomeren Klebeverbindungen definiert. Außerhalb der Klebeverbindungen liegen die Stofflagen lose aufeinander.

In der EP 2 822 520 B1 wird ein Verfahren zur Herstellung von Bandagen beschrieben, bei dem ein Elastomer im noch nicht abgebundenen Zustand auf eine elastische Stofflage schichtweise aufgetragen wird, wobei das Elastomer nach dem Abbinden ein dreidimensionales Formteil bildet, welches über eine Stoffseite übersteht.

Aus der EP 3 172 977 B1 ist ein Verfahren zur Herstellung von Bekleidungsstücken oder Bandagen bekannt, wobei eine Stofflage mit einem Verbund von Elastomer und Verstärkungsmaterial versehen wird. Der Verbund wird dadurch erzeugt, dass auf eine Elastomerschicht das Verstärkungsmaterial im flüssigen oder weichen, verformbaren Zustand aufgetragen wird, das nach dem Auftragen aushärtet. Die Elastomerschicht und das Verstärkungsmaterial werden jeweils über eine Düse aufgetragen.

Die DE 10 2017 119 328 A1 offenbart ein textiles Trägermaterial für ein Bekleidungsstück wie beispielsweise einen Büstenhalter, bei dem ein Elastomer auf mindestens zwei Abschnitte mindestens einer Stofflage des Trägermaterials aufgetragen ist. Das Elastomer ist innerhalb jedes Abschnitts jeweils in einem gleichmäßigen Elastomermuster punkt-, linien- oder flächenförmig auf die Stofflage aufgetragen, wobei sich der Elastomerauftrag zwischen den Abschnitten unterscheidet.

Der Erfindung liegt die Aufgabe zugrunde, ein Bekleidungsstück oder eine Bandage mit zwei übereinanderliegenden Stofflagen zu schaffen, wobei das Bekleidungsstück bzw. die Bandage sich durch einen hohen Tragekomfort und eine funktionale Anpassung der Stützkräfte auszeichnet.

Diese Aufgabe wird erfindungsgemäß mit den Merkmalen des Anspruches 1 gelöst. Die Unteransprüche geben zweckmäßige Weiterbildungen an.

Das mithilfe des erfindungsgemäßen Verfahrens hergestellte Bekleidungsstück bzw. die Bandage weist zwei übereinanderliegende Stofflagen auf, die mittels einer Elastomerverbindung teilweise miteinander verbunden sind. Das Elastomer, das für die Verbindung eingesetzt wird, kann hierbei punkt-, linien- oder flächenförmig aufgetragen werden, um die beiden Stofflagen miteinander zu verbinden. Der Auftrag des Elastomers erfolgt vorzugsweise in einem flüssigen oder weichen, verformbaren Zustand, in welchem das Elastomermaterial zumindest teilweise in die Stofflagen eindringt. Nach dem Aushärten des Elastomers besteht eine feste Verbindung zwischen dem Elastomer und jeder Stofflage, wobei aufgrund der Elastizität des Elastomers eine Nachgiebigkeit sowohl orthogonal zur Stofflagenebene als auch in beide Richtungen innerhalb der Stofflagenebene gegeben ist.

Bei einem punktförmigen Auftrag von Elastomer werden lediglich einzelne Elastomerpunkte skizziert, wohingegen bei einem linienförmigen Auftrag das Elastomer entlang einer geraden Linie oder einer gekrümmten Linie, gegebenenfalls einer abgeknickten Linie aufgetragen wird. Des Weiteren ist es auch möglich, einen flächigen Auftrag von Elastomer durchzuführen, beispielsweise rechteckförmig oder kreisförmig oder dergleichen. Es kann gegebenenfalls zweckmäßig sein, ein definiertes Muster von Elastomer aufzubringen, um gezielt auf die stützenden und tragenden Eigenschaften des herzustellenden Bekleidungsstücks bzw. der Bandage Einfluss zu nehmen. In jedem Fall erfolgt der Elastomerauftrag zum Verbinden der beiden Stofflagen jedoch nur teilweise, so dass nur ein Teil der aufeinanderliegenden Stofflagenflächen über das Elastomer miteinander verbunden sind und ein weiterer Teil elastomerfrei ist und lose aufeinanderliegt. Hierbei kann es ausreichend sein, dass weniger als die Hälfte der aufeinanderliegenden Flächen mit verbindendem Elastomer versehen ist.

Das Aufbringen von Elastomer zum Verbinden der beiden Stofflagen erfolgt in einem Verfahrensschritt. In einem weiteren Verfahrensschritt wird eine der Stofflagen durch schichtweisen Auftrag von Elastomer mit einem dreidimensionalen Formteil versehen, das über die Stofflage nach außen übersteht. Aufgrund der Verwendung von Elastomer weist auch das dreidimensionale Formteil ein hohes Maß an Elastizität auf. Für das Erzeugen des dreidimensionalen Formteils wird Elastomer schichtweise aufgetragen, wobei während des Auftrags das Elastomer sich noch im flüssigen oder weichen, verformbaren Zustand befindet und im Anschluss auf das Auftragen jeder Schicht das Elastomer aushärtet. Nach dem Aushärten kann eine weitere Schicht von Elastomer aufgetragen werden, bis das dreidimensionale Formteil seine gewünschte endgültige Form erreicht hat.

Das dreidimensionale Formteil haftet nur an der dieses Formteil tragenden Stofflage an. Vorteilhafterweise ist nur eine der beiden Stofflagen mit einem oder mehreren dreidimensionalen Formteilen versehen. Beim Aufbringen des Elastomers des dreidimensionalen Formteils in der ersten Schicht kann das Elastomermaterial in das Stofflagenmaterial eindringen; es erfolgt aber keine Verbindung zu der zweiten, darunterliegenden Stofflage. Das dreidimensionale Formteil befindet sich vorzugsweise an der Außenseite einer Stofflage und ist somit von der weiteren Stofflage abgewandt.

Bei dem dreidimensionalen Formteil werden in vorteilhafter Weise mehrere Schichten Elastomer aufgetragen. Grundsätzlich genügt es aber, nur eine einzige Elastomerschicht für die Herstellung des dreidimensionalen Formteils aufzutragen. Es ist zweckmäßig, dass das dreidimensionale Formteil eine größere Dicke - senkrecht zur Stofflagenebene gesehen - als die Elastomerverbindung aufweist.

Das dreidimensionale, elastische Formteil besteht zusätzlich zu der Elastomerverbindung zwischen den beiden Stofflagen. Das dreidimensionale Formteil kann insbesondere nach dem Anziehen oder Anlegen des Bekleidungsstücks bzw. der Bandage in gezielter und gewünschter Weise einen Druck auf eine bestimmte Stelle der das Bekleidungsstück oder die Bandage tragenden Person ausüben. Mithilfe dieses Drucks, der bezogen auf die Gesamtfläche des Bekleidungsstücks bzw. der Bandage lokal erhöht ist, kann dadurch beispielsweise eine massierende, eine in besonderer Weise stützende oder eine anderweitige medizinisch unterstützende Funktion erreicht werden. Im Falle eines Unterbekleidungsstücks wie beispielsweise einem Büstenhalter können über den gezielten Einsatz von dreidimensionalen Formteilen im Büstenhalter den Lymphfluss unterstützende Funktionen erzeugt werden. Das Bekleidungsstück bzw. die Bandage kann hierdurch auch medizinischen Zwecken dienen.

Das Aufbringen der Elastomerverbindung und das Aufbringen des dreidimensionalen Formteils erfolgt in unterschiedlichen Schritten, die zeitlich aufeinanderfolgen und/oder örtlich versetzt erfolgen. In bevorzugter Ausführung liegen die Schritte zeitlich aufeinander, wobei entweder die erste Elastomerverbindung und anschließend das dreidimensionale Formteil erzeugt werden oder bei umgekehrter Reihenfolge zunächst das dreidimensionale Formteil und anschließend die Elastomerverbindung erzeugt werden.

Bei räumlich oder örtlich verteilten, unterschiedlichen Schritten kann gegebenenfalls auch zeitlich überlappend bzw. gleichzeitig die Erzeugung der Elastomerverbindung sowie die Erzeugung des dreidimensionalen Formteils erfolgen.

Die Elastomerverbindung besteht aus mindestens einem verbindenden Elastomer in Punkt-, Linien- oder Flächenform, vorzugsweise aber aus mehreren derartigen Elastomerverbindungen. In dem weiteren Schritt wird mindestens ein dreidimensionales, elastisches Formteil erzeugt, vorzugsweise mehrere derartige elastische Formteile. Innerhalb des Schrittes zur Erzeugung der Elastomerverbindung können mehrere derartige Elastomerverbindungen entweder zeitlich aufeinanderfolgend oder zeitgleich hergestellt werden. Auch innerhalb des Schrittes zum Erzeugen mehrerer dreidimensionaler Formteile können diese entweder gleichzeitig oder zeitlich aufeinanderfolgend hergestellt werden.

Es kann zweckmäßig sein, für die Elastomerverbindung und für das dreidimensionale Formteil das gleiche Elastomermaterial zu verwenden. In alternativer Ausführung ist es auch möglich, unterschiedliche Elastomere für die Elastomerverbindung und das dreidimensionale Formteil einzusetzen. Als Elastomer kommt beispielsweise ein Silikon oder ein thermoplastisches Elastomer in Betracht.

Gemäß einer vorteilhaften Ausführung befinden sich ein Teil oder sämtliche Elastomerverbindungen und dreidimensionale Formteile an unterschiedlichen Positionen, bezogen auf die Fläche der übereinanderliegenden Stofflagen. Das Bekleidungsstück bzw. die Bandage kann beispielsweise in der Weise hergestellt werden, dass in einem bestimmten Teil oder Abschnitt die aufeinanderliegenden Stofflagen über mehrere Elastomerverbindungen verbunden sind, wobei ein oder mehrere dreidimensionale Formteile beabstandet zu den Elastomerverbindungen angeordnet sind, so dass eine Verteilung von Elastomerverbindungen und dreidimensionalen Formteilen in der Ebene der Stofflagen stattfindet, in der sich die Stofflagen ausbreiten. Für einen weiteren Abschnitt oder Teil des Bekleidungsstücks oder der Bandage kann es auch zweckmäßig sein, dass Elastomerverbindungen und dreidimensionale Formteile übereinanderliegen. Dementsprechend schneidet eine Senkrechte zur Stofflagenebene, die durch ein dreidimensionales Formteil geführt ist, eine darunterliegende Elastomerverbindung.

Beispielsweise kann es für einen Büstenhalter zweckmäßig sein, dass der Cup-Bereich Elastomerverbindungen und örtlich versetzt dreidimensionale Formteile aufweist. Für den Randbereich der Cups sowie für die Träger kann es dagegen zweckmäßig sein, ein streifenförmiges dreidimensionales Formteil entlang des Randes bzw. der Träger in einer durchgehenden Linie aufzubringen, wobei die beiden Stofflagen unter dem streifenförmigen Formteil über Elastomerverbindungen miteinander verbunden sind.

Gemäß noch einer weiteren zweckmäßigen Ausführung werden die Elastomerverbindung und das dreidimensionale Formteil auf unterschiedliche Weise hergestellt. So kann es beispielsweise zweckmäßig sein, die Elastomerverbindung im Siebdruckverfahren herzustellen. Alternativ kommen auch andere Auftragungstechniken in Betracht, beispielsweise Hochdruck, Tiefdruck, Rouleaux (Tiefdruckverfahren, bei dem gravierte Druckwalzen das Elastomer auf den Stoff auftragen), Sprühverfahren, Aufbringen über Düsen oder dergleichen.

Das Elastomer des dreidimensionalen Formteils wird zweckmäßigerweise über eine Düse aufgetragen. Hierbei können mehrere aufeinanderfolgende Schichten von Elastomer über die Düse aufgebracht werden. Alternativ zu einem Düsenauftrag kommen auch andere Auftragungstechniken in Betracht.

Gemäß noch einer weiteren vorteilhaften Ausführung wird das dreidimensionale Formteil als ein Verbund von Elastomer und einem Verstärkungsmaterial hergestellt. Elastomer und Verstärkungsmaterial werden im nicht ausgehärteten Zustand aufgetragen und härten nach dem Auftrag aus. Dies erfolgt insbesondere in der Weise, dass zunächst eine Elastomerschicht in einem weichen, nicht ausgehärteten Zustand aufgetragen wird, woraufhin das Verstärkungsmaterial im ebenfalls weichen, verformbaren Zustand auf die erste Elastomerschicht aufgetragen wird. Nach dem Aushärten des Verstärkungsmaterials kann gegebenenfalls eine weitere Elastomerschicht aufgetragen werden, um ein vollständiges Bedecken des Verstärkungsmaterials zu erreichen.

Vorteilhafterweise erfolgen sowohl der Auftrag der einen oder mehrerer Elastomerschichten für das dreidimensionale Formteil als auch der Auftrag des Verstärkungsmaterials über jeweils eine Düse. Die Position der Düsen kann in allen drei Raumrichtungen einstellbar sein, außerdem ist die Menge des über die jeweilige Düse austretenden Elastomermaterials und Verstärkungsmaterials jeweils einstellbar.

Als Verstärkungsmaterial kommt insbesondere ein Material in Betracht, das einen höheren Schmelzpunkt als das Elastomer aufweist, welches für das dreidimensionale Formteil verwendet wird. Es kann beispielsweise ein Kunststoffmaterial verwendet werden, zum Beispiel PLA (Polylactid)oder ABS (AcrylnitrilButadien-Styrol-Copolymerisat). Der Schmelzpunkt des Kunststoffmaterials liegt beispielsweise bei 180° oder 220°.

Es kann zweckmäßig sein, ein Verstärkungsmaterial zu wählen, dessen Biegsamkeit höher ist seine Dehnbarkeit. Dies ermöglicht es, das Verstärkungsmaterial raupenförmig oder streifenförmig in dem Verbund mit dem Elastomer aufzubringen, wobei entlang der Längserstreckung der Raupe bzw. des Streifens das Verstärkungsmaterial nur eine verhältnismäßig geringe Dehnbarkeit aufweist, was es ermöglicht, verhältnismäßig hohe Kräfte in der Längsrichtung der Raupe oder des Streifens aufzunehmen. Dagegen ist quer zur Längserstreckung der Raupe bzw. des Streifens eine gute Biegbarkeit des Verstärkungsmaterials gegeben. Dementsprechend eignet sich das Verstärkungsmaterial insbesondere für die Verwendung in einem Verbund mit dem Elastomer für einen Träger, beispielsweise eines Büstenhalters, und/oder im Randbereich eines Bekleidungsstücks oder einer Bandage. Aufgrund der hohen Biegbarkeit quer zur Längserstreckung ist ein hoher Tragekomfort gewährleistet.

In dem Verbund von Elastomer und Verstärkungsmaterial kann auch das Elastomer raupen- oder streifenförmig aufgetragen werden.

Gemäß noch einer weiteren vorteilhaften Ausführung wird das Elastomer streifenförmig auf eine der Stofflagen aufgetragen, wobei nach dem Aushärten in dem Elastomerstreifen die Stofflagen zur Herstellung eines Randbereichs des Bekleidungsstücks oder der Bandage geschnitten werden. Auch in dieser Ausführung bildet das Elastomer ein dreidimensionales Formteil, das über die Stofflage, auf die das Elastomer streifenförmig aufgetragen wird, nach außen übersteht. Zur Erzeugung eines Bügels oder Trägers kann es zweckmäßig sein, wie vorbeschrieben zusätzlich ein Verstärkungsmaterial einzubringen. In dieser Verwendung als Träger gibt es darüber hinaus den Vorteil, dass keine zusätzlichen Bauelemente erforderlich sind. Die Träger bestehen lediglich aus den beiden übereinanderliegenden und über Elastomerverbindungen verbundene Stofflagen und die zusätzliche, streifenförmige Elastomerschicht, die das dreidimensionale Formteil bildet und über eine Stofflage übersteht, gegebenenfalls mit integriertem Verstärkungsmaterial, über das erhöhte Längskräfte übertragbar sind.

Weitere Vorteile und zweckmäßige Ausführungen sind den weiteren Ansprüchen, der Figurenbeschreibung und den Zeichnungen zu entnehmen. Es zeigen:
- Fig. 1: einen Büstenhalter mit zwei übereinanderliegenden Stofflagen, die im Cup-Bereich über Elastomerpunkte miteinander verbunden sind, wobei zusätzlich im Cup-Bereich dreidimensionale und über die Stofflagenebene überstehende Formteile aus Elastomer angeordnet sind,
- Fig. 2: einen Schnitt durch den Büstenhalter im Cup-Bereich,
- Fig. 3: einen Schnitt durch einen Träger des Büstenhalters.

In den Figuren sind gleiche Bauteile mit gleichen Bezugszeichen versehen.

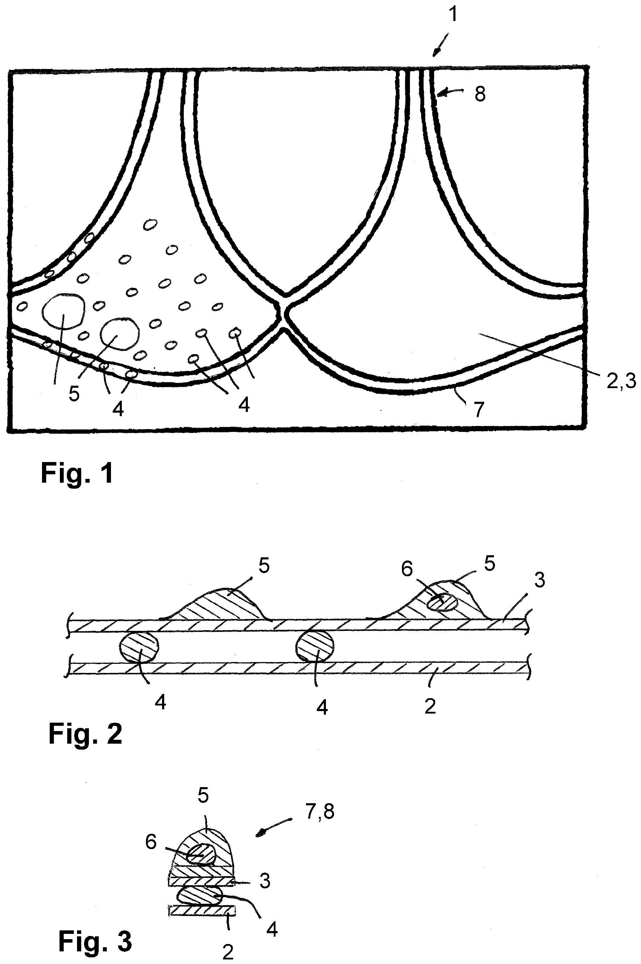

Wie Fig. 1 in Verbindung mit der Schnittdarstellung gemäß Fig. 2 zu entnehmen, besteht ein als Büstenhalter ausgeführtes Bekleidungsstück 1 aus zwei aufeinanderliegenden, elastischen Stofflagen 2, 3, die im Cup-Bereich und im Randbereich über Elastomerverbindungen 4 miteinander verbunden sind. In Fig. 1 ist exemplarisch nur ein Cup-Bereich mit Elastomerauftrag dargestellt; der zweite Cup-Bereich ist aber in gleicher Weise wie der erste Cup-Bereich mit Elastomerauftrag versehen.

Die Elastomerverbindungen 4 bestehen aus einzelnen Elastomerpunkten, die insbesondere im Siebdruckverfahren erzeugt und auf eine der beiden Stofflagen 2, 3 aufgebracht werden. Nach dem Aufbringen des Elastomers im weichen, verformbaren Zustand kann unmittelbar die zweite Stofflage 3 aufgesetzt werden, woraufhin das Elastomer aushärtet. Im weichen, formbaren Zustand dringt Elastomermaterial in jede Stofflage 2, 3 ein, so dass nach dem Aushärten eine feste Verbindung zwischen den beiden Stofflagen 2, 3 besteht, die aufgrund der Elastizität des Elastomermaterials eine elastische Relativbewegung zwischen den Stofflagen 2, 3 erlaubt.

Im Cup-Bereich des Büstenhalters 1 befinden sich eine Vielzahl von punktförmigen Elastomerverbindungen 4, die zueinander beabstandet sind. Es kann zweckmäßig sein, in verschiedenen Bereichen des Cups unterschiedliche Dichteverteilungen von Elastomerpunkten 4 vorzusehen, um hierdurch gezielt auf die tragenden und stützenden Eigenschaften Einfluss zu nehmen. Des Weiteren ist es möglich, anstelle eines punktförmigen Auftrags von Elastomermaterial alternativ, gegebenenfalls zusätzlich einen linien- oder flächenförmigen Auftrag von Elastomer zur Herstellung von Elastomerverbindungen 4 vorzusehen.

Im Cup-Bereich befinden sich mit Abstand zu den Elastomerverbindungen 4 beispielhaft zwei dreidimensionale Formteile 5, die ebenfalls aus einem Elastomermaterial bestehen, vorzugsweise aus dem gleichen Elastomer wie die Elastomerverbindungen 4. Wie der Schnittdarstellung gemäß Fig. 2 zu entnehmen ist, befinden sich die dreidimensionalen Formteile 5 auf der Außenseite der oberen Stofflage 3 und sind ausschließlich mit der oberen Stofflage 3 verbunden. Die dreidimensionalen Formteile weisen aufgrund ihrer Herstellung aus Elastomer ebenfalls ein elastisches Verhalten auf. Die dreidimensionalen Formteile 5 werden durch schichtweises Auftragen von Elastomer auf die obere Stofflage 3 erzeugt. Hierbei kann vorzugsweise über eine Düse das Elastomermaterial im nicht-ausgehärteten, weichen und formbaren Zustand in Schichten aufgetragen werden, wobei vorteilhafterweise zunächst die aufgetragene Schicht aushärtet und erst anschließend eine weitere Schicht aus Elastomermaterial aufgetragen wird.

Wie Fig. 2 zu entnehmen ist, ist in ein dreidimensionales Formteil 5 ein Verstärkungsmaterial 6 eingebracht. Das Verstärkungsmaterial 6 bildet gemeinsam mit dem Elastomermaterial das dreidimensionale Formteil als Verbund aus zwei Werkstoffen. Das Verstärkungsmaterial ist insbesondere ein Kunststoffmaterial, das ebenfalls über eine Düse aufgebracht werden kann, wobei das Verstärkungsmaterial im weichen, nicht ausgehärteten Zustand ausgebracht wird. Nach dem Ausbringen härtet das Verstärkungsmaterial aus. Anschließend kann eine weitere Schicht Elastomermaterial aufgebracht werden, so dass das Verstärkungsmaterial 6 vollständig von dem Elastomermaterial umschlossen ist.

Der Randbereich 7, welcher die Cup-Bereiche umrandet, kann wie in Fig. 3 dargestellt zum einen zwischen den beiden Stofflagen 2, 3 punktförmige Elastomerverbindungen 4 aufweisen, mit denen die beiden Stofflagen 2, 3 verbunden sind, und zum andern eine streifenförmige Elastomerschicht auf der oberen Stofflage 3 aufweisen, die nach Art eines dreidimensionalen Formteils 5 streifenförmig ausgebildet ist. Der Elastomerstreifen 5 wird der Kontur des Cup-Bereich folgend auf die obere Stofflage 3 aufgebracht, nach dem Aushärten des Elastomers kann in dem Elastomerstreifen zur Herstellung des Randbereichs 7 geschnitten werden. Hierdurch kann die gewünschte Büstenhalterform erreicht werden.

Auch die Träger 8 können in entsprechender Weise hergestellt sein. Hier kann es zusätzlich zweckmäßig sein, dass das dreidimensionale Formteil 5 ein Verstärkungsmaterial 6 aufweist, das raupenförmig ausgebildet ist und sich in Längsrichtung der Träger 8 erstreckt. Das Verstärkungsmaterial 6 besitzt eine verhältnismäßig geringe Dehnbarkeit in Längsrichtung, zugleich aber eine hohe Biegsamkeit quer zur Längserstreckung, so dass einerseits hohe Längskräfte über das Verstärkungsmaterial 6 aufgenommen werden können und andererseits aufgrund der Anpassung quer zur Längserstreckung ein hoher Tragekomfort gewährleistet ist.

Auch in dem den Cup-Bereich einfassenden Randbereich 7 kann in das dreidimensionale Formteil 5 gegebenenfalls ein Verstärkungsmaterial 6 eingebracht sein. In alternativer Ausführung ist es aber auch möglich, in diesem Randbereich 7 auf Verstärkungsmaterial zu verzichten.

## Patentansprüche

1. Verfahren zur Herstellung von Bekleidungsstücken oder Bandagen, mit zwei übereinanderliegenden Stofflagen (2, 3), wobei in zwei unterschiedlichen Schritten zum einen die beiden übereinanderliegenden Stofflagen (2, 3) teilweise mittels einer punkt-, linien- oder flächenförmigen Elastomerverbindung (4) verbunden werden und zum andern auf einer Stofflage (3) durch schichtweisen Auftrag von Elastomer ein dreidimensionales, elastisches und über die Stofflage (3) überstehendes Formteil (5) erzeugt wird, wobei das dreidimensionale Formteil (5) nur an der das Formteil tragenden Stofflage (3) anhaftet.

2. Verfahren nach Anspruch 1,
**dadurch gekennzeichnet,**
**dass** ein Teil oder sämtliche Elastomerverbindungen (4) und dreidimensionale Formteile (5) sich an unterschiedlichen Positionen befinden, bezogen auf die Fläche der übereinanderliegenden Stofflagen (2, 3).

3. Verfahren nach Anspruch 2,
**dadurch gekennzeichnet,**
**dass** die Elastomerverbindung (4) und das dreidimensionale Formteil (5) auf unterschiedliche Weise hergestellt werden.

4. Verfahren nach Anspruch 3,
**dadurch gekennzeichnet,**
**dass** die Elastomerverbindung (4) im Siebdruckverfahren hergestellt wird.

5. Verfahren nach Anspruch 3 oder 4,
**dadurch gekennzeichnet,**
**dass** das Elastomer des dreidimensionalen Formteils (5) über eine Düse aufgetragen wird.

6. Verfahren nach einem der Ansprüche 1 bis 5,
**dadurch gekennzeichnet,**
**dass** das dreidimensionale Formteil (5) als ein Verbund von Elastomer und einem Verstärkungsmaterial (6) hergestellt wird, wobei Elastomer und Verstärkungsmaterial (6) im nicht ausgehärteten Zustand aufgetragen werden und nach dem Auftrag aushärten.

7. Verfahren nach Anspruch 6,
**dadurch gekennzeichnet,**
**dass** die Biegsamkeit des Verstärkungsmaterials (6) höher ist als seine Dehnbarkeit.

8. Verfahren nach Anspruch 6 oder 7,
**dadurch gekennzeichnet,**
**dass** das Elastomer und das Verstärkungsmaterial (6) jeweils als Materialraupe aufgetragen werden.

9. Verfahren nach einem der Ansprüche 1 bis 8,
**dadurch gekennzeichnet,**
**dass** das Elastomer streifenförmig aufgetragen wird und anschließend nach dem Aushärten in dem Elastomerstreifen die Stofflagen (2, 3) zur Herstellung eines Randbereichs des Bekleidungsstücks (1) oder der Bandage geschnitten werden.

10. Verfahren nach einem der Ansprüche 1 bis 9,
**dadurch gekennzeichnet,**
**dass** ein oder mehrere dreidimensionale Formteile (5) senkrecht zur Ebene der Stofflagen (2, 3) eine größere Dicke aufweisen als die Elastomerverbindungen (4).

11. Bekleidungsstück oder Bandage, hergestellt nach dem Verfahren nach einem der Ansprüche 1 bis 10, wobei sich das dreidimensionale Formteil (5) auf der innenliegenden Stofflage (2) des Bekleidungsstücks (1) oder der Bandage befindet und die innenliegende Stofflage (2) bei Gebrauch auf der dem Körper zugewandten Seite der Person liegt, die das Bekleidungsstück (1) oder die Bandage trägt.

12. Büstenhalter als Bekleidungsstück nach Anspruch 11,
**dadurch gekennzeichnet,**
**dass** die Träger (8) des Büstenhalters (1) als ein Verbund von Elastomer und Verstärkungsmaterial (6) hergestellt sind.

## Claims

1. A method for producing garments or bandages, with two superimposed layers of fabric (2, 3), in two different steps, on the one hand, the two superimposed layers of fabric (2, 3) partially by means of a point, line or area-shaped elastomer connection (4) and on the other hand, a three-dimensional, elastic molded part (5) that protrudes over the fabric layer (3) is produced on the fabric layer (3) by layer-by-layer application of elastomer, the three-dimensional molded part (5) only being attached to the fabric layer (3).

2. The method according to claim 1, **characterized in that** part or all of the elastomeric compounds (4) and three-dimensional molded parts (5) are located at different positions in relation to the surface of the superimposed layers of fabric (2, 3).

3. The method according to claim 2, **characterized in that** the elastomer connection (4) and the three-dimensional molded part (5) are produced in different ways.

4. The method according to claim 3, **characterized in that** the elastomer connection (4) is produced by screen printing.

5. The method according to claim 3 or 4, **characterized in that** the elastomer of the three-dimensional molded part (5) is applied via a nozzle.

6. The method according to any one of claims 1 to 5, **characterized in that** the three-dimensional molded part (5) is produced as a composite of elastomer and a reinforcement material (6), with the elastomer and reinforcement material (6) being applied in the uncured state and curing after application.

7. The method according to claim 6, **characterized in that** the flexibility of the reinforcement material (6) is higher than its extensibility.

8. The method according to claim 6 or 7, **characterized in that** the elastomer and the reinforcing material (6) are each applied as a bead of material.

9. The method according to any one of claims 1 to 8, **characterized in that** the elastomer is applied in strips and then, after curing in the elastomer strip, the fabric layers (2, 3) are cut to produce an edge region of the item of the garment (1) or the bandage.

10. The method according to any one of claims 1 to 9, **characterized in that** one or more three-dimensional molded parts (5) perpendicular to the plane of the fabric layers (2, 3) have a greater thickness than the elastomer connection (4).

11. Garment or bandage, produced by the method according to any one of claims 1 to 10, wherein the three-dimensional molded part (5) is on the inner fabric layer (2) of the garment (1) or the bandage and the inner fabric layer (2) at use is on the side facing the body of the person wearing the garment (1) or bandage.

12. Brassiere as a garment according to claim 11, **characterized in that** the straps (8) of the brassiere (1) are produced as a composite of elastomer and reinforcement material (6).

## Revendications

1. Procédé de réalisation de vêtements ou bandages, à deux couches de tissu superposées (2, 3), en deux étapes différentes, d'une part, les deux couches de tissu superposées (2, 3) partiellement au moyen d'une liaison élastomère (4) ponctuelle, linéaire ou surfacique et d'autre part, une pièce moulée élastique tridimensionnelle (5) qui dépasse de la couche de tissu (3) est produite sur une couche de tissu (3) par application par couche d'une couche d'élastomère, la pièce moulée tridimensionnelle (5) n'étant fixée qu'à la couche de tissu (3) fixée.

2. Procédé selon la revendication 1, **caractérisé en ce qu'**une partie ou la totalité des liaisons élastomères (4) et des pièces moulées tridimensionnelles (5) sont situées à des positions différentes par rapport à la surface des couches de tissu superposées (2, 3).

3. Procédé selon la revendication 2, **caractérisé en ce que** la liaison élastomère (4) et la pièce moulée tridimensionnelle (5) sont fabriquées de différentes manières.

4. Procédé selon la revendication 3, **caractérisé en ce que** la liaison élastomère (4) est réalisée par sérigraphie.

5. Procédé selon la revendication 3 ou 4, **caractérisé en ce que** l'élastomère de la pièce moulée tridimensionnelle (5) est appliqué via une buse.

6. Procédé selon l'une quelconque des revendications 1 à 5, **caractérisé en ce que** la pièce moulée tridimensionnelle (5) est réalisée sous la forme d'un composite d'élastomère et d'un matériau de renforcement (6), l'élastomère et le matériau de renforcement (6) étant appliqués à l'état cru et durcissant après l'application.

7. Procédé selon la revendication 6, **caractérisé en ce que** la flexibilité du matériau de renforcement (6) est supérieure à son extensibilité.

8. Procédé selon la revendication 6 ou 7, **caractérisé en ce que** l'élastomère et le matériau de renforcement (6) sont chacun appliqués sous la forme d'un cordon de matériau.

9. Procédé selon l'une quelconque des revendications 1 à 8, **caractérisé en ce que** l'élastomère est appliqué en bandes puis, après durcissement dans la bande d'élastomère, les couches de tissu (2, 3) sont coupées pour produire une zone de bordure du vêtement (1) ou du bandage.

10. Procédé selon l'une quelconque des revendications 1 à 9, **caractérisé en ce qu'**une ou plusieurs pièces moulées tridimensionnelles (5) perpendiculaires au plan des couches de tissu (2, 3) ont une épaisseur plus importante que les liaisons élastomères (4).

11. Vêtement ou bandage, réalisé par le procédé selon l'une quelconque des revendications 1 à 10, dans lequel la pièce moulée tridimensionnelle (5) est sur la couche intérieure de tissu (2) du vêtement (1) ou du bandage et la couche intérieure de tissu (2) à l'usage est du côté faisant face au corps de la personne portant le vêtement (1) ou le bandage.

12. Soutien-gorge en tant que vêtement selon la revendication 11, **caractérisé en ce que** les bretelles (8) du soutien-gorge (1) sont réalisées en un composite d'élastomère et de matériau de renforcement (6).
